# EUROPÄISCHE PATENTSCHRIFT

(11) **EP 1 430 018 B1**
(45) Veröffentlichungstag und Bekanntmachung des Hinweises auf die Patenterteilung: **29.12.2010**
(21) Anmeldenummer: 02767473.8
(22) Anmeldetag: 04.09.2002
(51) Int. Cl.: C07C 209/48, C07C 279/14, C07C 237/02, C07D 239/20

(54) **VERFAHREN ZUR HERSTELLUNG VON BETA-ALANINAMIDEN**
METHOD FOR PRODUCING BETA-ALANINE AMIDES
PROCEDE DE PRODUCTION D'AMIDES DE BETA-ALANINE

(30) Priorität: 06.09.2001 EP 01121342; 26.11.2001 US 332547 P
(43) Veröffentlichungstag der Anmeldung: 23.06.2004
(73) Patentinhaber: Lonza AG, 4052 Basel (CH)
(72) Erfinder: HANSELMANN, Paul, CH-3902 Glis (CH); HILDBRAND, Stefan, CH-4322 Mumpf (CH)
(86) Internationale Anmeldenummer: PCT/EP2002/009893
(87) Internationale Veröffentlichungsnummer: WO 2003/022795

(56) Entgegenhaltungen:
- EP-A- 0 294 668
- WO-A-01/64638
- WO-A-91/05555
- US-A- 4 359 416
- DATABASE CROSSFIRE BEILSTEIN [Online] Beilstein Institut zur Förderung der Chemischen Wissenschaften, Frankfurt am Main, DE; retrieved from XFIRE Database accession no. br 222197 XP002224290 & ANDREAE, S.; SCHMITZ, E.; WULF, J.; SCHULZ, B.: LIEBIGS ANN. CHEM., Bd. 3, 1992, Seiten 239-256,
- DATABASE CROSSFIRE BEILSTEIN [Online] Beilstein Institut zur Förderung der Chemischen Wissenschaften, Frankfurt am Main, DE; retrieved from XFIRE Database accession no. br 395823 XP002224291 & SCHAPER, W.: SYNTHESIS, Bd. 9, 1985, Seiten 861-867,
- DATABASE CROSSFIRE BEILSTEIN [Online] Beilstein Institut zur Förderung der Chemischen Wissenschaften, Frankfurt am Main, DE; retrieved from XFIRE Database accession no. br 774851 XP002224292 & COSSEY, A. L. ET AL.: AUST. J. CHEM., Bd. 29, 1976, Seiten 1039-1050,
- NORMAN R O C: "Principles of Organic Synthesis" , PRINCIPLES OF ORGANIC SYNTHESIS, PAGE(S) 572 , LONDON XP002172715 das ganze Dokument
- DATABASE CROSSFIRE BEILSTEIN [Online] Beilstein Institut zur Förderung der Chemischen Wissenschaften, Frankfurt am Main, DE; retrieved from XFIRE Database accession no. rid 630250 XP002224293 & OSDENE, T. S. ET AL.: J. MED. CHEM., Bd. 10, 1967, Seiten 165-171, & SHUKLA, J. S.; BHATIA, P.: J. INDIAN CHEM. SOC., Bd. 55, 1978, Seiten 281-283,
- DATABASE CROSSFIRE BEILSTEIN [Online] Beilstein Institut zur Förderung der Chemischen Wissenschaften, Frankfurt am Main, DE; retrieved from XFIRE Database accession no. rid 663077 XP002224294 & LEONARD; BOYER: J. AMER. CHEM. SOC., Bd. 72, 1950, Seiten 2980-2984,
- DATABASE CROSSFIRE BEILSTEIN [Online] Beilstein Institut zur Förderung der Chemischen Wissenschaften, Frankfurt am Main, DE; retrieved from XFIRE Database accession no. rid 68295 XP002224295 & BOWMAN; CAVALLA: J. CHEM. SOC., 1954, Seiten 1171-1176,

## Beschreibung

Die Erfindung betrifft ein Verfahren zur Herstellung von Amiden des β-Alanins, insbesondere von Pseudo-Dipeptiden wie beispielsweise Carcinin. Sie betrifft weiterhin neue Cyanessigsäureamide als Zwischenprodukte im erfindungsgemässen Verfahren.

Carcinin (β-Alanyl-histamin) ist ein natürlich vorkommendes Pseudo-Dipeptid der nachstehenden Struktur, welches aus tierischem Gewebe isoliert werden kann. Carcinin hat eine antioxidative Wirkung und ist als Wirkstoff gegen gewisse Komplikationen des Diabetes (US-A-5561110), insbesondere Katarakt (US-A-5792784), sowie als Bestandteil von kosmetischen Zubereitungen (US-B-6280715) vorgeschlagen worden.

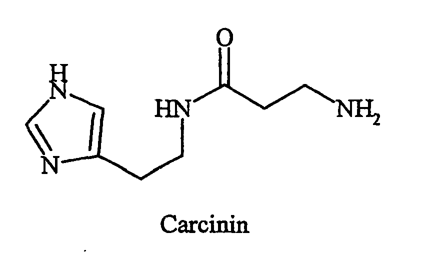

Die bekannten Synthesen von Carcinin aus den Bestandteilen Histamin und β-Alanin erfordern den Einsatz von Schutzgruppen und/oder aktivierten Derivaten und sind deshalb für die kostengünstige Herstellung grosser Mengen wenig geeignet (EP1020179).

EP-A-0294668 offenbart die Umsetzung von Cyanessigsäuremethylester mit Aminosäureester-hydrochloriden zu *N*-Cyanacetyl-aminosäureestern.

G. A. Swan (J. Chem. Soc. 1950, 1539-1542) sowie G. Blaskó et al. (G. Blaskó, E. Major, G. Blaskö, I. Rözsa und C. Szäntay, Eur. J. Med. Chem. Chim. Ther. 1986, 21, 91-95) beschreiben die Herstellung von Cyanessigsäure-(2-indol-3-yl-ethylamid) (= *N*-Cyanacetyl-tryptamin) (CrossFire Beilstein Database, Registry Nr. 222197) aus 2-Indol-3-yl-ethylamin (Tryptamin) und Cyanessigsäureethylester.

J. Sam (J. Pharm. Sci. 1967, 56, 1202-1205) beschreibt die Herstellung von 2-Cyan-*N*-(2-pyridin-2-yl-ethyl)acetamid (CrossFire Beilstein Database, Registry Nr. 395823) aus 2-(Pyridin-2-yl)ethylamin und Cyanacetylchlorid.

Y. Otsuji et al. (Y. Otsuji, N. Matsumura und E. Imoto, Bull. Chem. Soc. Jpn. 1968, 41, 1485) sowie A. L. Cossey et al. (A. L. Cossey, R. L. N. Harris, J. L. Huppatz und J. N. Phillips, Aust. J. Chem. 1976, 29, 1039-1050) beschreiben die Herstellung von *N*-*n*-Hexylcyanacetamid (CrossFire Beilstein Database, Registry Nr. 774851) aus Cyanessigsäureethylester und *n*-Hexylamin. Y. Otsuji et al. beschreiben ausserdem die analoge Herstellung von *N*-*n*-Hexadecylcyanacetamid während A. L. Cossey et al. unter anderem die analoge Herstellung von *N*-*n*-Octylcyanacetamid und *N*-Isopropylcyanacetamid beschreiben.

Aufgabe der vorliegenden Erfindung war daher, ein für die technische Synthese von Carcinin und anderen Pseudo-Dipeptiden des β-Alanins geeignetes Verfahren bereitzustellen, das ohne die Verwendung von Schutzgruppen oder teuren Derivaten auskommt und nur gut zugängliche Ausgangsmaterialien einsetzt.

Erfindungsgemäss wird diese Aufgabe durch das Verfahren nach Patentanspruch 1 gelöst.

Die erfindungsgemäss herstellbaren β-Alaninamide besitzen die allgemeine Formel

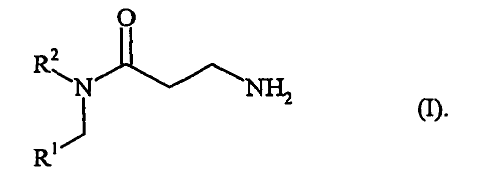

Hierin bedeutet R¹ gegebenenfalls mit C₁₋₆-Alkyl substituiertes Imidazol-4-ylmethyl und R² ist Wasserstoff.

Diese Verbindungen könnnen in neutraler Form oder - nach Protonierung der primären Aminogruppe - als Salze mit Säuren vorliegen. Die Verbindungen, die Imidazolylreste enthalten, können auch in mehreren tautomeren Formen oder als Gemisch solcher Formen vorliegen.

Unter C₁₋₆-Alkyl sind hier und im folgenden alle linearen oder verzweigten primären, sekundären oder tertiären Alkylgruppen mit 1 bis 6 Kohlenstoffatomen zu verstehen, also beispielsweise Methyl, Ethyl, Propyl, Isopropyl, Butyl, *sec*-Butyl, *tert*-Butyl, Isobutyl, Pentyl, Isopentyl, Neopentyl, 2-Methylbutyl, Hexyl, 2-Methylpentyl oder 3-Methylpentyl. Entsprechendes gilt für C₁₋₁₀-Alkyl, wozu neben den bereits genannten beispielsweise noch Gruppen wie Octyl oder 2-Ethylhexyl zählen.

Es wurde gefunden, dass Amine der allgemeinen Formel 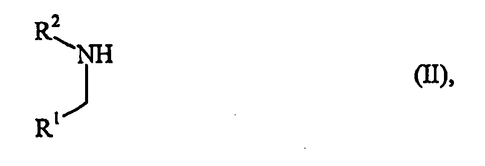 worin R¹ und R² die oben genannten Bedeutungen haben, mit einem Cyanessigsäureester der allgemeinen Formel 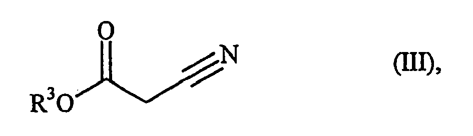 worin R³ C₁₋₁₀-Alkyl ist, zu einem Cyanessigsäureamid der allgemeinen Formel 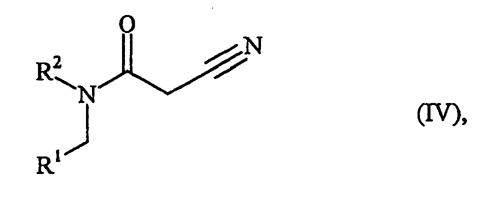 worin R¹ und R² die oben genannten Bedeutungen haben, oder einem entsprechenden Salz umgesetzt werden können und das Cyanessigsäureamid (IV) durch katalytische Hydrierung in die Zielverbindung (I) oder ein entsprechendes Salz übergeführt werden kann.

Besonders bevorzugt ist R¹ Imidazol-4-ylmethyl oder 3-Methylimidazol-4-ylmethyl.

R³ ist vorzugsweise Methyl oder Ethyl.

Falls das Amin (II) als Salz vorliegt, in dem die primäre Aminogruppe protoniert ist, muss diese zunächst durch Zugabe einer Base deprotoniert werden. Als Base sind hierbei grundsätzlich alle Basen verwendbar, die eine stärkere Basizität als die primäre Aminogruppe aufweisen. Vorzugsweise wird eine mittelstarke bis starke Base eingesetzt. Hierfür eignen sich beispielsweise Alkalihydroxide wie Natrium- oder Kaliumhydroxid, tertiäre Amine wie Triethylamin, 4-Dimethylaminopyridin, 1,4-Diaza[2.2.2]bicyclooctan, bicyclische Amidine ("DBN", "DBU") sowie in nichtwässrigen Lösungsmitteln Alkalialkoholate wie Natriummethanolat oder -ethanolat und in aprotischen Lösungsmitteln auch Alkalimetallhydride und -amide wie beispielsweise Natriumhydrid oder -amid. Die Base wird vorzugsweise in stöchiometrischer oder annähernd stöchiometrischer Menge eingesetzt.

Bevorzugte Lösungsmittel für die erste Stufe sind polare protische oder aprotische Lösungsmittel wie Wasser, C₁₋₄-Alkanole wie beispielsweise Methanol oder Ethanol, und Amide wie beispielsweise *N*,*N*-Dimethylformamid, *N*,*N*-Dimethylacetamid, 1-Methyl-2-pyrrolidon oder 1,3-Dimethyl-3,4,5,6-tetrahydro-2(1*H*)-pyrimidinon (DMPU).

Als Katalysatoren für die Hydrierung werden vorzugsweise Metallkatalysatoren auf Basis von Nickel, Cobalt, Kupfer, Rhodium, Palladium, Ruthenium oder Platin eingesetzt, die gegebenenfalls auf einem Träger aufgezogen sind. Hierzu zählen beispielsweise Nickel- und Cobaltkatalysatoren vom Raney-Typ, feinverteiltes Platin (z. B. durch Reduktion von PtO₂ erhalten), Rhodium, Palladium oder Platin auf Aktivkohle oder Aluminiumoxid oder Cobalt auf Siliciumdioxid (Silica).

Besonders bevorzugt sind Raney-Nickel und Raney-Cobalt sowie Rhodium auf Aktivkohle oder Aluminiumoxid.
Als Lösungsmittel in der Hydrierung können die bei der Hydrierung von Nitrilen zu Aminen üblichen Lösungsmittel wie beispielsweise Wasser, konzentrierte wässrige Ammoniaklösung, Methanol, Ethanol, *N*,*N*-Dimethylformamid oder Gemische der genannten Lösungsmittel eingesetzt werden.

Die Cyanessigsäureamide der allgemeinen Formel 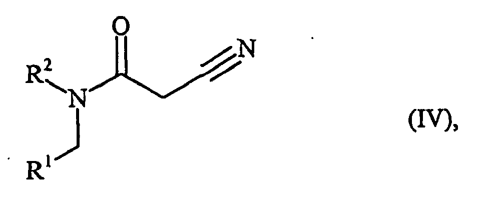 worin R¹ und R² die oben genannten Bedeutungen haben, sowie deren Salze sind neu und ebenfalls Gegenstand der Erfindung.

Besonders bevorzugte Cyanessigsäureamide (IV) sind *N*-(Cyanacetyl)-histamin der Formel 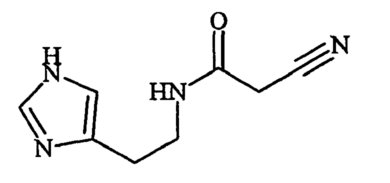 und dessen Salze und Tautomere sowie *N*-(Cyanacetyl)-3-methyl-histamin der Formel 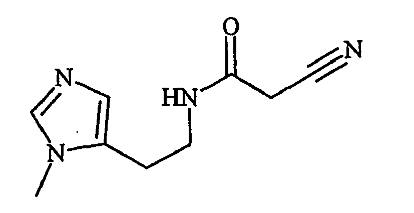 sowie dessen Salze.

Die folgenden Beispiele verdeutlichen die Durchführung des erfindungsgemässen Verfahrens und die Herstellung der erfindungsgemässen Verbindungen.

### Beispiel 1

### 2-Cyan-N-[2-(1(3)H-imidazol-4-yl)ethyl]acetamid (N-Cyanacetylhistamin)

Zu einer Lösung von 5,00 g (45 mmol) Histamin in 50 g Ethanol wurden bei 60 °C 7,60 g (67,2 mmol) Cyanessigsäureethylester getropft. Nach 2 h bei 80 °C wurde das Reaktionsgemisch am Rotationsverdampfer eingeengt und der Rückstand mittels Flash-Säulenchromatographie an Kieselgel (Elutionsmittel: Gradient Ethylacetat → Ethylacetat/Methanol 1:1) gereinigt.
Ausbeute: 6,61 g (82%)
¹H NMR (DMSO-*d*₆, 400 MHz): δ = 11,8 (br., 1H); 8,28 (t, 1H); 7,52 (s, 1H); 6,80 (s, 1H); 3,60 (s, 2H); 3,30 (q, 2H); 2,65 (t, 2H).
¹³C NMR (DMSO-*d*₆, 100 MHz): δ = 161,92; 134,63; 134,21; 116,64; 116,13; 39,25; 26,63; 25,23.
LC-MS: *m*/*z* =179 ([M+H]⁺), 161, 149, 138, 112, 95, 83.

### Beispiel 2

### 3-Amino-N-[2-(1H-imidazol-4-yl-ethyl]propionamid (β-Alanylhistamin, Carcinin)

Zu einer Lösung von 1,00 g (5,61 mmol) 2-Cyan-*N*-[2-(1(3)*H*-imidazol-4-yl)ethyl]acetamid (hergestellt gemäss Beispiel 1) in 11,4 g Ethanol und 7,6 g konzentrierter wässriger Ammoniaklösung wurden 92 mg Rh/Al₂O₃ (5% Rh) gegeben. Das Gemisch wurde bei 90 °C und 50 bar Wasserstoffdruck 2 h hydriert. Der Katalysator wurde über Celite^{®} abfiltriert und das Filtrat am Rotationsverdampfer eingeengt. Nach längerem Trocknen bei 50 °C/20 mbar wurden 0,89 g rohes Carcinin mit einem Gehalt (HPLC) von 73% erhalten.
¹H NMR (DMSO-*d*₆, 400 MHz): δ = 12 (br., 1H); 7,95 (t, 1H); 7,50 (s, 1H); 6,77 (s, 1H); 3,6 (br., 1H); 3,25 (q, 2H); 2,74 (t, 2H); 2,63 (t, 2H); 2,15 (t, 2H).
LC-MS: *m*/*z* = 183 ([M+H]⁺), 166, 148, 124, 95.

## Patentansprüche

1. Verfahren zur Herstellung von β-Alaninamiden der allgemeinen Formel 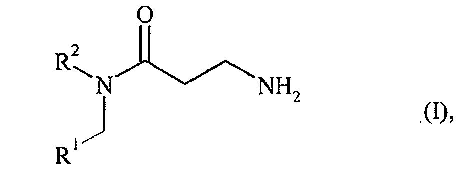 worin R¹ gegebenenfalls mit C₁₋₆-Alkyl substituiertes Imidazol-4-ylmethyl und R² Wasserstoff ist,
**dadurch gekennzeichnet, dass** ein Amin der allgemeinen Formel 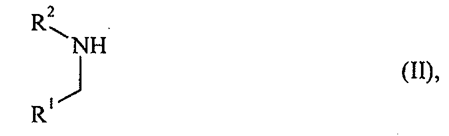 worin R¹ und R² die oben genannten Bedeutungen haben, oder ein entsprechendes Salz in einer ersten Stufe mit einem Cyanessigsäureester der allgemeinen Formel 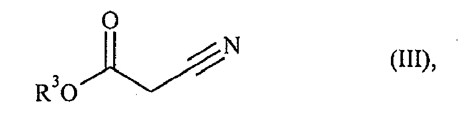 worin R³ C₁₋₁₀-Alkyl ist, zu einem Cyanessigsäureamid der allgemeinen Formel 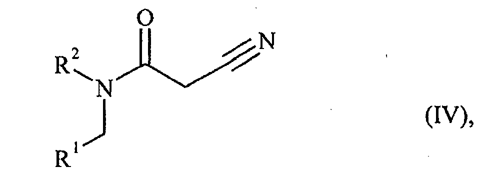 worin R¹ und R² die oben genannten Bedeutungen haben, oder einem entsprechenden Salz umgesetzt wird, welches in einer zweiten Stufe durch katalytische Hydrierung in die Zielverbindung (I) oder ein entsprechendes Salz übergeführt wird.

2. Verfahren nach Anspruch 1, **dadurch gekennzeichnet, dass** als Lösungsmittel in der ersten Stufe Ethanol und/oder *N,N*-Dimethylformamid eingesetzt wird.

3. Verfahren nach Anspruch 1 oder 2, **dadurch gekennzeichnet, dass** als Katalysator in der zweiten Stufe ein Katalysator aus der Gruppe bestehend aus Rhodium auf Aktivkohle, Rhodium auf Aluminiumoxid, Raney-Nickel und Raney-Cobalt eingesetzt wird.

4. Cyanessigsäureamide der allgemeinen Formel 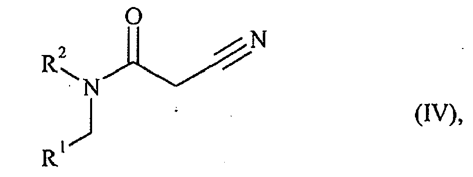 worin R¹ und R² die in Anspruch 1 genannten Bedeutungen haben, sowie deren Salze.

5. *N*-(Cyanacetyl)-histamin der Formel 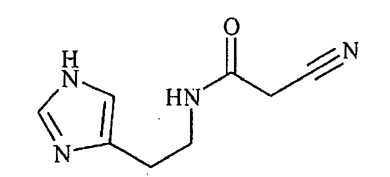 sowie dessen Salze und Tautomere.

6. *N*-(Cyanacetyl)-3-methyl-histamin der Formel 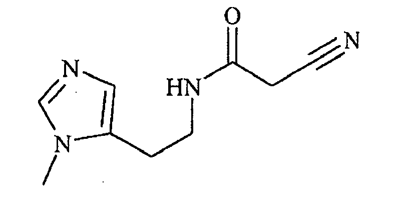 sowie dessen Salze.

## Claims

1. Method for preparing β-alaninamides of the general formula 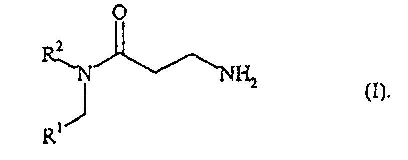 in which R¹ is unsubstituted or C₁₋₆-alkyl- substituted imidazol-4-ylmethyl and R² is hydrogen,
**characterized in that** an amine of the general formula 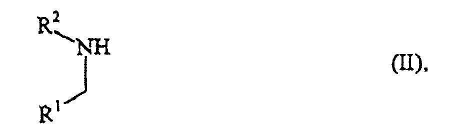 in which R¹ and R² are as defined above, or a corresponding salt is reacted in a first stage with a cyanoacetic ester of the general formula 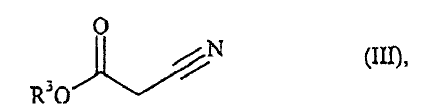 in which R³ is C₁₋₁₀-alkyl, to give a cyanoacetamide of the general formula 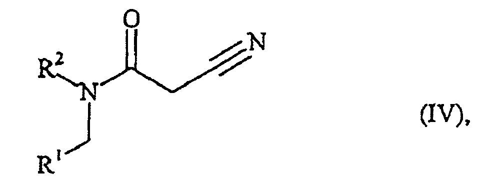 in which R¹ and R² are as defined above, or a corresponding salt, which, in a second stage, is converted to the target compound (I) or a corresponding salt by catalytic hydrogenation.

2. Method according to Claim 1, **characterized in that** the solvent used in the first stage is ethanol and/or *N*,*N*-dimethyl-formamide.

3. Method according to Claim 1 or 2, **characterized in that** the catalyst used in the second stage is a catalyst from the group consisting of rhodium on activated carbon, rhodium on aluminum oxide, Raney nickel and Raney cobalt.

4. Cyanoacetamides of the general formula 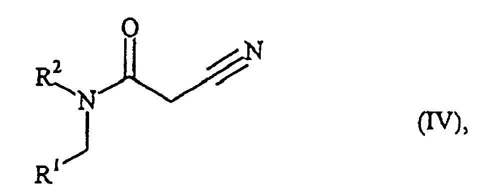 in which R¹ and R² are as defined in Claim 1, and salts thereof.

5. N-(Cyanoacetyl)histamine of the formula 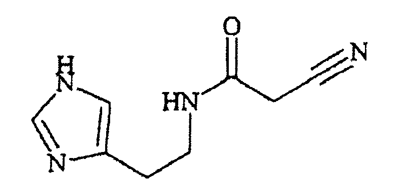 and its salts and tautomers.

6. *N*-(Cyanoacetyl)-3-methylhistamine of the formula 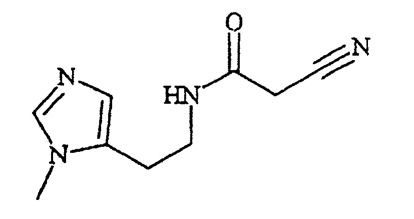 and its salts.

## Revendications

1. Procédé pour la préparation de β-alaninamides de formule générale 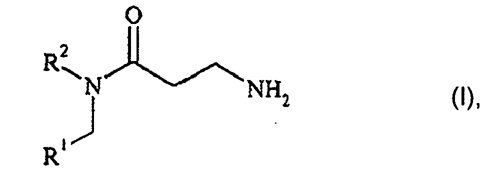 dans laquelle R¹ est un groupe imidazol-4-ylméthyle éventuellement substitué par alkyle en C₁-C₆ et R² est un atome d'hydrogène,
**caractérisé en ce qu'**on fait réagir une amine de formule générale 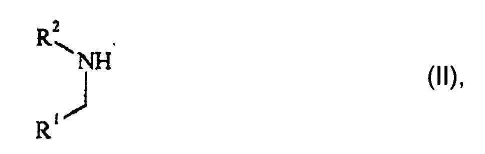 dans laquelle R¹ et R² ont les significations données ci-dessus, ou un sel correspondant, dans une première étape, avec un ester d'acide cyanoacétique de formule générale 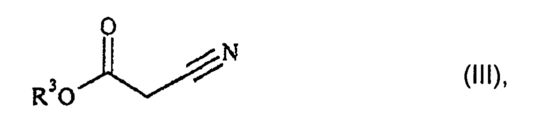 dans laquelle R³ est un groupe alkyle en C₁-C₁₀. pour aboutir à un cyanoacétamide de formule générale 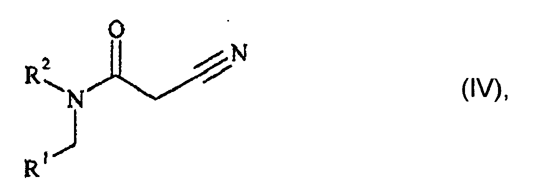 dans laquelle R¹ et R² ont les significations données ci-dessus, ou un sel correspondant, qui est converti, dans une deuxième étape, par hydrogénation catalytique, en le composé (I) recherché ou en un sel correspondant.

2. Procédé selon la revendication 1, **caractérisé en ce qu'**on utilise comme solvant dans la première étape l'éthanol et/ou le N,N-diméthylformamide.

3. Procédé selon la revendication 1 ou 2, **caractérisé en ce qu'**on utilise comme catalyseur dans la deuxième étape un catalyseur choisi dans le groupe constitué par le rhodium sur charbon actif, le rhodium sur oxyde d'aluminium, le nickel de Raney et le cobalt de Raney.

4. Cyanoacétamides de formule générale 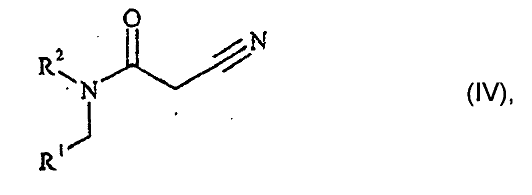 dans laquelle R¹ et R² ont les significations données dans la revendication 1, ainsi que leurs sels.

5. N-(cyanoacétyl)-histamine de formule 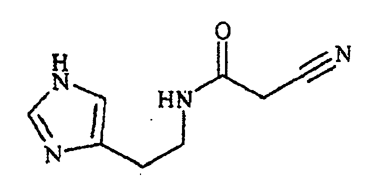 ainsi que ses sels et tautomères.

6. N-(cyanoacétyl)-3-méthyl-histamine de formule 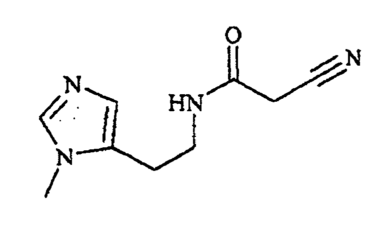 ainsi que ses sels.
